# EUROPEAN PATENT SPECIFICATION

(11) **EP 1 398 047 B1**
(45) Date of publication and mention of the grant of the patent: **16.11.2005**
(21) Application number: 02020305.5
(22) Date of filing: 11.09.2002
(51) Int. Cl.: A61M 1/16, A61M 1/36, A61M 1/10, B01D 63/02

(54) **End-cap for a dialyser, dialyser and method for removing gas bubbles**
Kappe für einen Dialysator, Dialysator und Verfahren zur Entfernung von Gasblasen
Couvercle pour un dialyseur, dialyseur et méthode pour enlever des bulles de gaz

(43) Date of publication of application: 17.03.2004
(73) Proprietor: Gambro Lundia AB, 220 10 Lund (SE)
(72) Inventor: Deppisch, Reinhold, 72379 Hechingen (DE); Hildwein, Helmut, 72072 Tübingen (DE)
(74) Representative: Harrison, Michael Charles

(56) References cited:
- EP-A- 0 355 325
- EP-A- 0 378 225
- EP-A- 0 534 386
- WO-A-02/078768
- DE-A- 10 017 690
- FR-A- 2 267 138
- US-A- 3 856 475

## Description

The present invention relates to a dialyser end-cap and to a dialyser, in particular a dialyser including such an improved end-cap, and to a method for removing gas bubbles within a dialyser.

### BACKGROUND TO THE INVENTION

Dialysers are used in dialysis for a variety of purposes. Examples include haemodialysis, in which a filter is provided in the form of a semi-permeable membrane, and blood is passed on one side of the semi-permeable membrane while dialysis fluid is passed on the other side of the membrane. Differing convection and diffusion processes hereby take place through the membrane, which bring about a cleaning of the blood as well as a removal of excess fluid. Additionally, the electrolyte concentrations in the blood are hereby conditioned, and buffers such as bicarbonate or acetate are added to the blood.

Such dialyser devices are also used in so-called haemofiltration, in which a substitution fluid is added to the blood. The blood is passed on one side of the semi-permeable membrane, but no dialysis fluid is passed on the other side of the semi-permeable membrane. Instead, inside the filter, excess fluid, in particular water, as well as waste products, is removed from the blood merely with the aid of a pressure difference across the semi-permeable membrane. The substitution fluid can be added to the blood either before or after the filtration.

The devices mentioned above may equally be used in order to produce the substitution fluid itself, these then being termed ultrafilters. In this case, dialysis fluid for example is passed on one side of the semi-permeable membrane and is filtered through this by means of a pressure difference across the semi-permeable membrane, whereby the dialysis fluid is sterile-filtered by removal of bacteria and endotoxins as well as other contamination products.

A further possibility for use of the mentioned dialysis devices is for example haemodiafiltration, a combination of haemodialysis and haemofiltration. The term dialyser, used herein, should be understood also to include filter devices comprising a housing and semi-permeable membranes which may be used for the purpose of plasmapheresis, in which the watery blood plasma is filtered out of the blood and after a treatment is supplied to the blood again. Such dialysers may also be used with reverse osmosis and therapeutic plasma exchange (TPE) as well as other similar treatment procedures. The general term "dialyser" used herein also includes haemofilters, ultrafilters and haemodiafilters.

The aforementioned dialysers are normally constructed such that a plurality of semi-permeable membranes, which are formed as flat membranes or as hollow fibres, are arranged in a housing. The housing comprises connections for supplying and removing blood as well as connections for the supply and removal of dialysis fluid, substitution fluid or ultrafiltrate respectively.

If semi-permeable hollow fibres are used, the hollow fibres are collected together as a loose bundle and arranged in a tubular housing. The tubular housing comprises an end cap at each end, whereby the hollow fibre bundle is arranged between the ends of the housing such that the end caps cover the respective ends of the hollow fibre bundle. Each of the ends of the hollow fibre bundle also additionally comprises an enclosing support ring and is potted into the ends of the housing by means of a potting compound.

The collected hollow fibres of the hollow fibre bundle open out with their respective open ends into an interior space formed as a hollow cavity and called a header chamber, between the end cap and the end of the hollow fibre bundle. In this way, by appropriate arrangement of the mentioned inlets and outlets, it is possible to provide, in a known manner, the various dialyser filter types such as dialysers for haemodialysis, haemofiltration, haemodiafiltration, ultrafiltration, etc.

Examples of the aforementioned dialysers are disclosed in EP-A-0 305 687, EP-A-0 355 325 and EP-A-0 525 317.

If one of the aforementioned filters is applied in the area of dialysis, for example in haemodialysis, differing fluid lines (conduits) are connected to it. These fluid lines on the one hand lead the blood of the patient to the blood side of the filter and from there back again to the patient, and on the other hand they lead the dialysis fluid from a dialysis fluid supply, controlled by a monitor to the dialysate side of the filter and from there on to a drain. The blood side here refers to the area of the filter through which the blood of the patient is lead, while the dialysate side refers to the area of the filter or the filter housing respectively, through which the dialysis fluid is lead. The blood side and the dialysate side are separated from each other in the filter housing by the semi-permeable membrane(s).

The blood-conducting lines, which together with the blood side of the filter form part of the extracorporeal blood circuit, are normally replaced together with the filter after each treatment, while the dialysis fluid-conducting lines remain connected to the dialysis monitor and are disinfected and flushed after each treatment.

The extracorporeal blood circuit understandably has to be precisely and reliably monitored, in order to be sure that under all circumstances any danger to the patient during the treatment is avoided. For monitoring the extracorporeal blood circuit, differing devices are therefore provided, these being referred to below as functional parts of the extracorporeal blood circuit, which inter alia monitor the venous and arterial pressure, which control blood flow, which de-gas the return or venous blood and which check the return blood for air or gas bubbles.

The blood flow is normally controlled by means of a peristaltic pump. This comprises means which continually occlude the line from the outside, along a region thereof, and thus create a flow in the line without coming into direct contact with the blood.

The degassing of the venous blood normally occurs in a drip chamber and the blood flowing out of the drip chamber is checked for air or gas bubbles with the aid of e.g. an air detector before return to the patient.

The monitoring of the arterial or venous pressure is done with the aid of sensors, which normally detect the arterial pressure upstream of the peristaltic pump and the venous pressure in the drip chamber.

The monitoring of the pressure during dialysis not only in the blood-conducting lines, but also in the dialysis fluid-conducting lines, is important for several reasons.

One important reason is for example the monitoring of the transmembrane pressure at the membrane in the dialyser, which as mentioned above separates the blood from the dialysis fluid and is semi-permeable. This transmembrane pressure is the pressure difference between the pressure on the blood side and the pressure on the dialysate side, and determines the direction and magnitude of the fluid transport through the membrane. If the transmembrane pressure is positive, fluid is extracted from the blood. This process is also called ultrafiltration.

With negative membrane pressures no excess fluid is extracted from the patient. The extraction of excess fluid is however one of the main tasks of dialysis, so that even this alone necessitates monitoring of the pressure in the dialysis fluid lines and the blood lines.

There exists another important reason for monitoring the arterial and venous pressure in the blood-conducting lines during treatment. If the arterial pressure value goes below a particular negative pressure, the arterial needle can become blocked or it can be stuck in the fistula wall, or the line could be bent. In order to avoid damage to the fistula, the machine is automatically stopped and an alarm given. If the arterial pressure exceeds a predetermined value, the arterial needle could have become dislodged and be sucking in air. Also here an alarm is given automatically and the machine stopped.

If on the other hand the venous pressure falls below a predetermined value, the blood return to the patient might not be sealed or could have been interrupted. If the venous pressure exceeds a particular predetermined value, the venous needle could be blocked or the return line could be bent. In these cases, the machine is also stopped and an alarm is automatically given.

It is here seen to be disadvantageous that the blood-conducting lines have to be adapted to the aforementioned functional parts of the extracorporeal blood circuit. For this, a specially formed arterial blood line as well as a specially formed venous blood line are required, whereby each of these has to be adapted to different functional parts. Due to the required flexibility and elasticity respectively of the blood lines and the complexity of the functional parts, this cannot be automated in the manufacture of arterial and venous blood lines. The compulsory manual operations thus required make the manufacture of the complicated arterial and venous blood lines time-consuming and cost-intensive.

For example, the arterial blood line needs, apart from the connection for the fistula needle, a specially formed connector piece for the arterial pressure sensor as well as a specially formed pump segment, which is placed in the peristaltic pump. Furthermore a connection for adding medicinal substances such as heparin is also required, as well as an additional connection for the removal of samples or other substances.

The venous blood line needs, apart from the connection for the fistula needle, a complexly formed drip chamber with several connections as well as a specially formed connector piece for the venous pressure sensor, in case this is not integrated into the drip chamber. Beyond this, a specially formed connector piece for the air detector may be necessary, according to which type of air detector is used in the monitor.

As already mentioned above, known dialysers are generally configured such that a tubular filter-housing encases a bundle of hollow-fibre membranes. The housing is provided with end-caps at either end, each end-cap forming part of a header-chamber at the opening of the fibre-bundle.

The tubular housing, as well as the end-caps of known dialyser devices are usually constructed from transparent polycarbonate or polypropylene material. Whilst these materials provide a high degree of rigidity and transparency to the device, there are nevertheless certain drawbacks associated with their use for this purpose.

Polycarbonate and polypropylene are considered to have a comparatively low degree of biocompatibility. It is therefore necessary to coat component parts with a more highly biocompatible layer before using them in a dialyser. This increases the unit cost of the parts and of the dialyser itself.

In addition, dialyser units are generally sterilised by a steam-treatment process. This treatment causes thermal expansion and contraction effects in the housing and end-cap components. When rigid materials are used, this treatment can lead to gaps occurring in joins between, for example, the end-caps and the tubular housing, where differences in expansion and contraction may have occurred. This occurs in particular as a result of the use of different polymeric materials for the various components, where the respective parts also have different structures and dimensions. In order to overcome these effects, it has become necessary to provide comparatively complex constructions including numerous additional gasketing means between the housing and end-cap means, in order to ensure a good seal between the ends of the capillary filter means, the housing, and the end-caps. Manufacturing tolerances are also required to be precise in order for the hitherto relatively rigid component parts to fit together well enough to provide a good seal. This further increases the component and assembly cost of such devices.

A further important aspect of known dialysis processes is the need to reduce the risk of thromboses or clots during a blood treatment. Since a haemodialysis treatment generally lasts for between three to six hours, the risk of thromboses or clots developing is substantial, especially towards the end of such a treatment. Thrombogeneicity becomes increased as a result of increased exposure of blood to air. The more functional parts there exist in a blood circuit, the greater is the volume of blood in the circuit and therefore the more time it takes for blood, flowing at a given rate, to flow through the circuit. This increases the exposure of blood to air.

For example, in a known manner, the blood flowing into a haemodialysis filter housing typically enters and exits the filter means through a header chamber provided at either end of the filter means. Known header chambers are provided with an internal generally conical form, in order to facilitate the escape of gas bubbles up the blood tube towards de-gas means. This conical shape however increases the volume of the chamber and also the time spent by blood passing through it, thereby increasing thrombogeneicity.

In order to reduce this problem, it is desirable to reduce the volume of the extracorporeal blood circuit and also to reduce the volume required for the functional elements around the said circuit, while nevertheless maintaining full functionality.

A further drawback of known blood filtration and dialysis devices is that the blood passing through the header chamber tends, over a period of time, to develop preferred flow paths. This can lead to uneven perfusion of blood through the hollow-fibre membrane filter, and can lead to stagnation of some blood in the header chamber, again increasing the risk of thromboses.

Against this background it is therefore an object of the present invention to avoid one or more of the various disadvantages mentioned in detail above.

### SUMMARY OF THE INVENTION

The present invention provides an end-cap having an end wall with a liquid inflow or outflow aperture and an attachment portion for attachment to an end portion of a dialyser, the end-cap being shaped such that it defines an interior region or space wherein a portion of the end wall is movable relative to the attachment portion. The volume of the interior region may thereby be changed.

The invention is defined in appended claim 1. Further features are defined in claims 2-31.

The said interior region defines a header chamber, when the end-cap is connected to a dialyser. The end-cap is shaped such that it has an attachment portion allowing secure attachment to a housing or other portion of a dialysis filter. A suitable attachment portion may for example be a peripheral, generally circular wall. The attachment means may additionally comprise mouldings for positive engagement with a dialyser housing and for generating a liquid-tight seal between the end-cap and the housing.

Additionally, the end-cap wall is provided with a liquid inflow or outflow aperture. The aperture is intended to allow the desired flow of liquids into or out of the interior region of the end-cap, in order to enable adequate passage of liquid, in particular blood, through the dialyser header-chambers, and filter means. The aperture may be combined in a single moulding or in more than one piece, with a blood tube forming part of an extracorporeal blood circuit.

The extent of the moveability of at least a part of the end-cap wall should be appreciable in regard to the dimensions of the end-cap. For example, a movement range of around 1-10mm or more is envisaged for this part, such that there may thereby be an appreciable volume change within the interior of the end cap when the movable part of the end-cap wall is displaced. In addition, the moveability should be such that any gas bubbles collected within the interior region of the end cap, or header-chamber, may be encouraged to escape, for example, through said aperture.

Such an arrangement may allow the provision of pressure equalisation means within an extracorporeal blood circuit to be dispensed with. Furthermore, the end-cap may thereby form part of a liquid pressure-monitoring device.

The moveable part of the end-wall may be provided in a number of ways. For example, a portion of the end-cap wall may be formed from excess material, capable of being extended in response to a force upon the end-cap wall. Alternatively, a portion of the end-wall may be comprised of a flexible material, in particular a material having elastic properties. In this way, the end-cap wall may be readily distorted in response to an external force, or, in response to a liquid pressure change within the end-cap interior region and will tend to revert to its original shape upon removal of distorting forces.

The present invention may be realised either as an end-cap, or as an end-cap and dialyser combination, wherein the end-cap is attached to a dialyser housing.

The present invention also provides an end-cap for a dialyser, the end-cap being made entirely from an elastic material. In addition, the invention provides a dialyser having an elastic end-cap as well as a method of removing gas bubbles from a dialyser having an elastic end-cap. The invention further provides a system for pumping blood around an extracorporeal blood circuit, wherein elastic end-caps on a dialyser filter-housing form part of a membrane pump.

Known prior art dialyser end-caps have been made from rigid polycarbonate material, which is also essentially non-elastic. The flexible portion, or in a preferred form, the entire end-cap according to the present invention can be made from a material having an elastic deformability of at least 5 % . Preferably, the elastic deformability of the material lies above 10%. It is also envisaged that the elasticity may lie above 20% . The invention may also be realised by using a flexible material for the end-cap having elasticity in the range between 10-30% . Preferably, the walls of the end-cap according to the invention have a thickness between 0.5-6mm, and are such that the end-cap possesses sufficient flexibility to readily deform in response to manipulation by a human operator using minimal force, e.g. fingertip force, or in response to pressure changes of liquids contained within the end-cap wall when in place upon a dialyser housing. The end-cap wall may have a different thickness at different points.

In this regard, the term "elastic" means that the material used will regain its initial shape after deformation within its elastic limit, with minimal permanent strain.

Such an end-cap, when in place upon a dialyser housing, may be readily manipulated by an operator during a treatment. Thus, a gentle massaging action on the end cap may be used to enhance the liquid flow within the header chamber in such a manner as to prevent the build-up of blood clots in areas of slower liquid-flow, in particular at the outer peripheral regions close to where the end-cap is connected to the housing.

The escape of any gas-bubbles collected within a header chamber may be assisted by providing the end-cap wall with a conical elevation angle. Such an angle may lie in the range from 0-30° or between 7-15°.

Alternatively, by simply raising the portion of the end-cap to which a blood tube is attached, an operator can cause any gas bubbles within the header chamber to move away up the blood tube to a de-gassing device. This effect could also be achieved by moving the dialyser or filter housing downward with respect to the end-cap or blood tube.

The possibility of removing gas bubbles in the way described reduces the need for, and may even remove the need for a conical interior shape of the header chamber. This in turn allows the chamber to be configured with a smaller interior volume, thereby reducing thrombogeneicity by decreasing the time taken for the blood to pass though the chamber. Thus, the end-walls of the end-cap can even be configured to be substantially perpendicular to the axis of the dialyser housing or filter housing.

Additionally, the provision of an end-cap having at least an attachment portion which is elastic, can allow considerably easier assembly of the dialysis filter by removing the problems arising from dimensional change of mating components during and after steam-sterilisation, and by making component fit tolerances less critical.

The flexible elastic end-cap or the flexible portion of the end-cap can be made from any suitable material, although the chosen material should preferably possess biocompatibility in addition to its elastic characteristics. Biocompatibility is important, since the elastic end-cap is in direct contact with blood. The elastic end-cap of the invention may thus be formed from a polymer with elastic properties, such as polyurethane or polyvinylchloride, or from materials based on polypropylene or polyethylene, which contain elastic polymers. Alternatively, silicone based compounds may be used.

Other materials envisaged for the end-cap or for the flexible portion of the end-cap of the present invention include polyurethane, which has an increased biocompatibility as compared with polycarbonate used hitherto. The need for costly coating of components can thereby also be removed. Further materials which are suitable include transparent polyolefins of various types, or materials such as SEBS or silicon for example.

The flexible and elastic property of the end-caps is also such that small changes in blood pressure inside the filter housing can be accommodated by a corresponding elastic deformation of the end-cap, thereby producing a volume change. This therefore increases the compliance of the system as a whole to the varying pressures encountered during operation. The need to incorporate pressure-equalising means in an extracorporeal blood circuit may therefore be removed.

In a further preferred embodiment it is envisaged that means for detecting the arterial and/or venous pressure are arranged on the elastic end-caps. This is advantageously achieved by the arrangement of a deformation sensor on the elastic end-cap(s), to detect the deformation caused by the change in arterial and/or venous pressures.

The deformation sensor can be formed in any suitable manner, as long as the deformation of the elastic end-cap caused by the arterial and/or venous pressure can be detected. It is however advantageous if the deformation sensor comprises strain measurement strips, which are known per se and are obtainable in many different forms. This reduces the manufacturing cost of the filter further and moreover simplifies the manufacture, since the strain measurement strips can easily be attached to the elastic end-cap, for example by adhesion. Alternatively, other suitable measurement means may be employed, such as mechanical displacement measurement means, or optical means for measuring material distortion or stress.

In a further embodiment, the flexible end-caps are set up to function as parts of a membrane type pump means for pumping blood through the filter and around a blood circuit. In particular, one or more of the end-caps may be driven in a reciprocating motion relative to the housing or vice-versa, in cooperation with means for opening and closing one or more blood lines connected to the respective header chambers. In this way, the pump means are effectively incorporated into the filter housing, thereby avoiding the need for provision of additional pump means.

Another embodiment is envisaged in which the end-cap is formed transparent. This provides the advantage that means for detecting air bubbles, which means can be provided in preferred embodiments, and which work according to the photoelectric principle, can be arranged on the end-cap.

The filter housing can have any form, although it is advantageous to form the housing tubular, and with a flexible end cap on each end. It is then preferred to arrange means for detecting the arterial pressure on one end cap and means for measuring the venous pressure on the other end cap. A bundle with semi-permeable hollow fibre membranes is preferably arranged in the tubular housing.

According to a further embodiment of the invention, a blood tube is formed integral with the end-cap, joined to the end-cap wall at the inflow/outflow aperture in the said wall. The length of the tube may be long (e.g. 1 m or more) or short, such that the tube may provide a connection means to an arterial or venous blood line, or may itself constitute a proportion of, or substantially all of, an arterial or venous blood line.

An arrangement where the blood tube is integrally formed with the end-cap may help reduce set-up time of the device, by reducing the number of blood-tube connectors necessary. Additionally, such an arrangement may simplify the set-up requirements for a dialyser, by removing the requirements for certain additional fittings between the end-cap and a blood line. The risk of contamination of the blood line may also thereby be reduced.

As can be appreciated form the present disclosure, there are many advantages associated with the use of a flexible end-cap for a dialyser, in particular a flexible and elastic end-cap. However, a further problem exists with known dialyser or semi-permeable membrane-type filter devices, in that the pressure profile across the membrane, along the axis of the housing, cannot be altered. The aforementioned pressure profile determines the relative extent of the migration of substances into or out of the blood.

In response to this limitation, a further development of the present invention (not claimed) provides a dialyser in which the tubular filter housing is made from a flexible material, in particular a flexible and elastic material, enabling the filtering characteristics within the filter-housing to be influenced by manipulating (e.g. by squeezing) the housing.

The invention is defined in the appended independent claims, with further preferred embodiments being defined in the dependent claims.

### BRIEF DESCRIPTION OF THE DRAWINGS

Fig. 1 illustrates a generally known dialyser and end-cap configuration,
Figs. 2a-b and 2c show alternative embodiments of end-caps according to the invention, whereby Figure 2a is a sectional view along line IIa-IIa and Figure 2b is a plan view taken along the line IIb-IIb,
Figs. 3a-c illustrate partial views of end-cap configurations in place upon a dialyser housing,
Figs. 4a and 4b show another embodiment of an end-cap attached to a filter or dialyser housing, and illustrate a functionality of the flexible elastic property of the end-cap of the invention,
Figs. 5a and 5b provide a schematic illustration of a configuration in which valve means and mechanical actuation means are set up such that the end-cap elastic portions function as parts of a membrane-pump for urging blood through the extracorporeal blood circuit and dialyser.
Fig. 6 illustrates development of the invention (not claimed) in which the filter housing itself is comprised of a flexible elastic material.
Fig. 7 illustrates the fluid pressure profile along the length of a known semi-permeable membrane filter, such as may be contained within a known dialyser.
Fig. 8 shows a further embodiment of the invention

### DESCRIPTION OF EMBODIMENTS

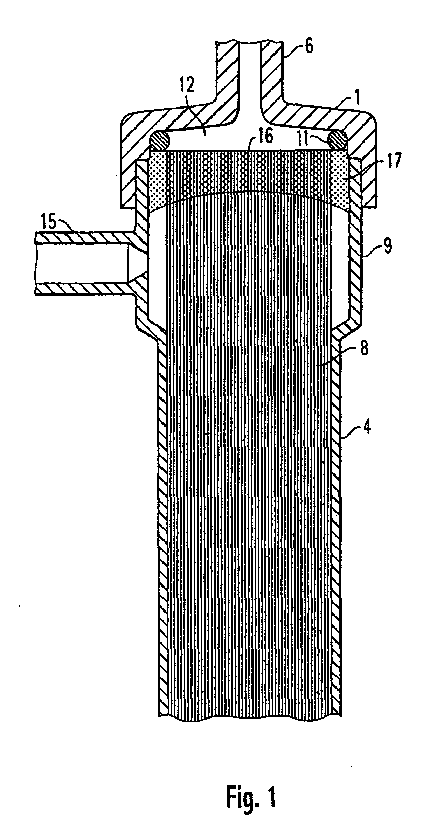

Figure 1 shows a dialyser housing and end-cap configuration generally known in the art, used for e.g. blood treatment such as dialysis, ultrafiltration or plasmapheresis. In particular, the illustration shows a membrane dialyser, specifically, a capillary fibre dialyser. In the case of a dialysis treatment, blood from the arterial blood line of an extracorporeal blood circuit is pumped into the filter for example through the tubular portion 6 which forms an aperture for liquid flow into the interior region of the end-cap. Since the end-cap is attached to the housing 4, a first header chamber 12 is formed into which the blood enters. From there, it passes through the hollow fibre semi-permeable membrane bundle 8, while dialysis or substitution fluid flows through the elongated casing, or housing 4 around the exterior of the hollow fibre membranes 8 in the opposite direction to the blood flow. The semi-permeable membrane thereby separates the blood flow passages from the dialysis fluid passages between the fibres. In a conventional manner, the blood exits from a header chamber at the far end (not shown) of the filter membranes, from where it enters a venous blood line (not shown). The dialysis or substitution fluid, exits the housing at the outlet 15, having entered through a similar tubular aperture (not shown) at the opposite end (not shown) of the housing 4. The hollow fibre bundle 8 is held in place at both ends of the dialyser housing 4 using a potting compound 16, the said potting compound being formed with a peripheral portion 17 for locating the end of the fibre bundle 8 within a widened end portion 9 of the housing 4. A ring-shaped seal 11 may be employed between the end-cap wall and the peripheral portion of the potting compound 17 to ensure against leakage of blood outside the header chamber 12.

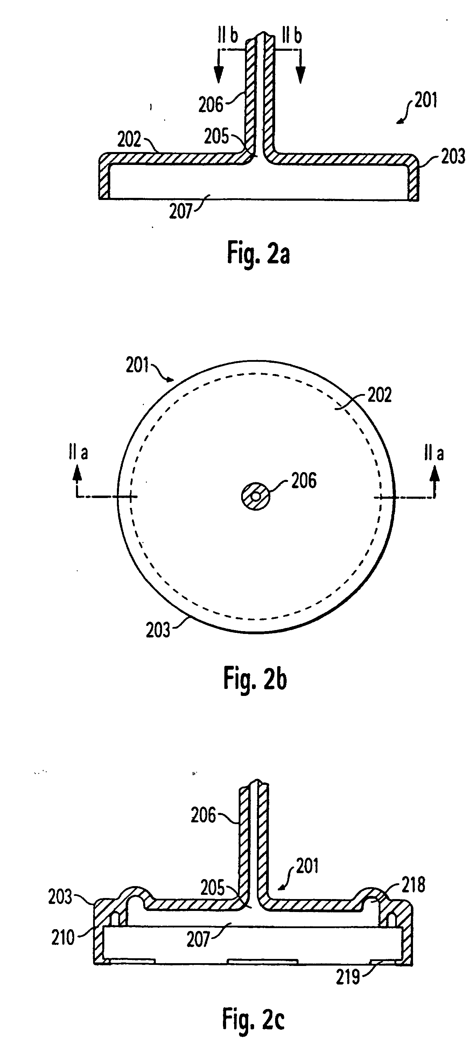

With reference to Figure 2a, a dialyser end-cap according to the invention is illustrated in a sectional view, indicated generally by the reference numeral 201. The end-cap 201 comprises a wall having a tubular inlet or outlet portion 206, a lateral end-wall 202 having an aperture 205 and a peripheral wall 203. The tubular portion 206 is preferably formed integral with the end-cap, more preferably, injection-molded in one-piece and from the same material as the end-cap. The arrangement of the peripheral wall is such that the end-cap defines a generally interior region 207, whereby the peripheral wall 203 is shaped to be capable of functioning as an attachment means, for secure, liquid-tight attachment to an end of a tubular dialyser filter-housing. The tubular member may have any desired length. Specifically, the tubular portion 206 may be configured as a short length, suitable for connection to a liquid-flow circuit via a connector means, or it may be configured with a length sufficient to constitute a substantial part of a liquid flow circuit, such as an arterial or venous blood line of an extracorporeal dialysis circuit.

Figure 2c shows a sectional view of an end-cap according to the invention similar to that shown in Figure 2a, in which the peripheral wall 203 is conformed to provide enhanced attachment and sealing properties when in place upon a dialyser filter housing. These may include an optional engagement portion 219 for improving the connection of the end-cap 201 to a housing. A recessed interior portion 218 allows the possibility of a sealing ring to be accommodated within a peripheral portion of the interior region of the end-cap. A further recessed portion 210 provides a means for secure attachment of the end-cap to e.g. a dialyser filter housing.

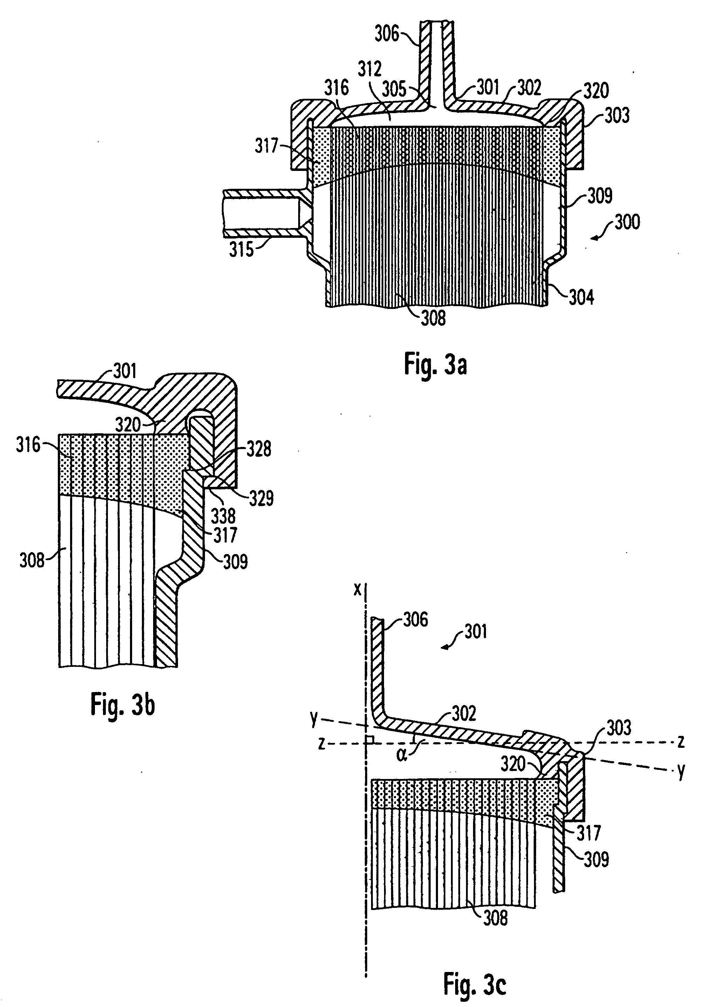

As can be seen from the embodiment shown in Figure 3a, the elastic end-cap 301 may be formed such that an internal shoulder 320 is provided internally of the peripheral wall 303. The internal shoulder may be formed with a flat or curved end surface for example. The elastic properties of the end-cap material thereby enable an effective seal preventing liquid leakage from the header chamber 312 to the outside of the filter or to the portion of the filter housing 304 containing dialysis fluid. The hollow fibres 308 are restrained at their open ends within a potting compound 316. As described in Figure 2a, there may usefully be formed a peripheral support portion 317 of the potting compound. This serves to accommodate and locate the ends of the hollow fibres 308 within the end portion 309 of the housing 304, the diameter of which is typically greater than the internal diameter of the main body of the tubular housing 304. In Figure 3a, secure engagement between the end-cap 301 and the end 309 of the housing 304 is shown, provided by means of friction between the cap peripheral portion 303 and the housing. However, it is to be understood that any suitable means of engagement or bonding may be employed, such as, for example by means of adhesive or welding techniques, e.g. by ultrasound welding. As shown in Figure 3b, the potting compound peripheral portion 317 may be shaped to co-operate with an internal rim 328 formed within the end 309 of the filter housing 304 in order to locate and support the hollow fibre end-portions. Additionally, Figure 3b illustrates a possibility for enhancing the seating of the end-cap 301 on the outside of the housing end-portion 309, by providing an external shoulder 329 for engagement with engagement portion 338.

Figure 3c illustrates a partial section of an end-cap 301 according to the invention, showing the internal conical angle α between the flat or generally conical end-cap lateral wall 302 and the line Z-Z, which lies perpendicular to the longitudinal axis of the dialyser, which is also parallel to the axis of rotation X-X of the end-cap and which is typically also parallel to the end-cap peripheral wall 303, when the end-cap 301 is in place on the dialyser housing end-portion 309. The line Y-Y represents the general inclination of the flexible lateral wall 302. According to the invention, the angle of elevation, i.e. the internal conical angle, of the end-cap lateral wall may lie between 0°-30°, preferablybetween 7°-15°.

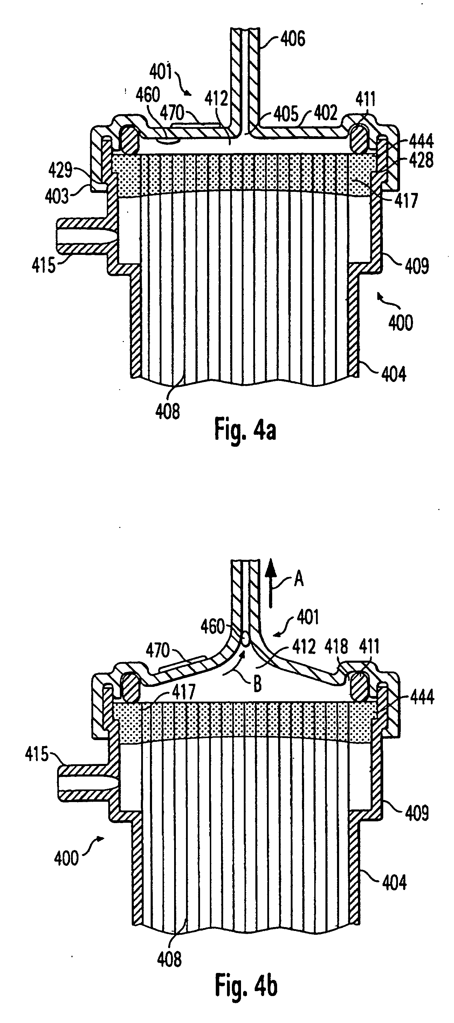

Figures 4a and 4b illustrate the flexible nature of the elastic end-cap 401 according to the invention, which allows the manipulation of the end-cap lateral wall 402 by a user or operator. This may be of assistance to fill the filter during priming, or to help maintain a homogenous perfusion of blood through the hollow fibres 408 during e.g. a dialysis process. In addition, as is illustrated in Fig.4b, part of the end-cap 401 may be moved in the direction of arrow A in such a manner as to encourage the displacement of air or gas bubbles 460 up the tube 406 and thereby out of the header chamber 412 along arrow B. The elastic properties of the flexible lateral end-wall 402 may be further utilised in order to monitor the liquid pressure within a header-chamber 412. This may be achieved, for example, by incorporating a strain-gauge 470 within or on the external surface of the end-wall 402. Other suitable deformation detection means may be used in accordance with the present invention, although these are not shown here. Also shown in Figures 4a and 4b is the end of the fibre bundle 408, embedded in the potting compound 416, 417 and surrounded by a ring-shaped support member 444, configured to co-operate with seating means 428 provided at the end of the filter housing 409 as is known per se. In the embodiment shown in Figures 4a and 4b, a sealing ring 411 provides a liquid-tight seal between the end-cap 401 and the end of the fibre bundle 408. The end-cap 401 could however equally be configured to create its own seal with the fibre bundle, for example, as shown in Figures 3a and 3b. A strain measurement means such as strain-gauge 470 is shown on the end-cap 401, in the form of a strain-gauge strip. Such a strip, when connected via connection means (not shown) to a measurement device, such as a resistance bridge circuit, and/or a control device (not shown), can thereby constitute means for monitoring liquid pressure within the header chamber 412. The measurement means 470 is shown attached to the surface of the end-cap, although it could equally be wholly or partially embedded within the material of the end-cap.

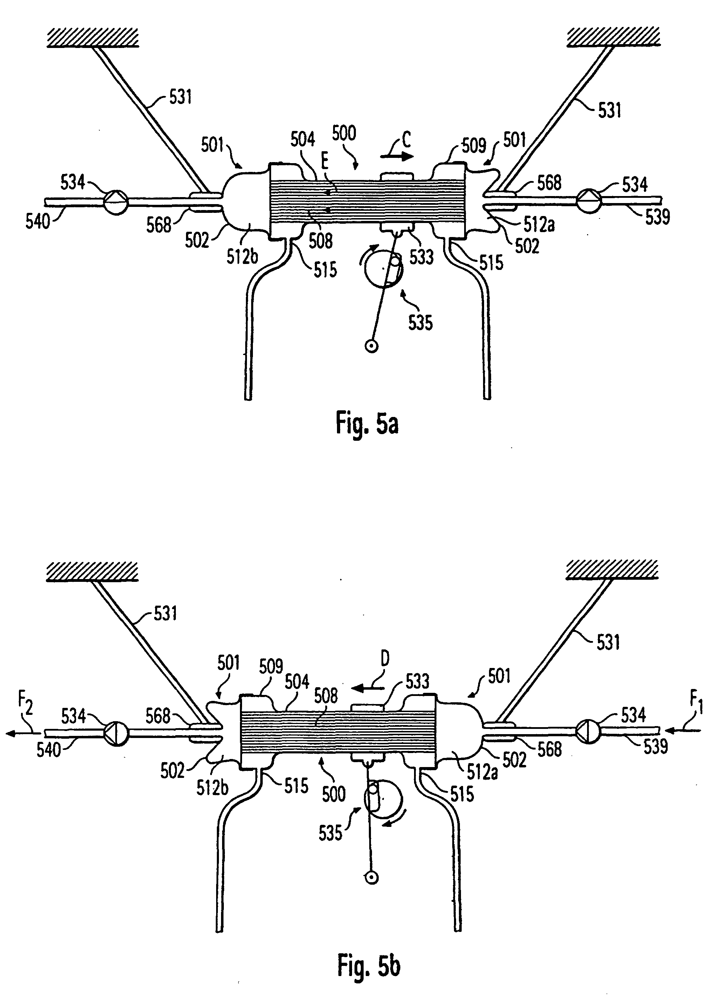

The diagram according to Figures 5a and 5b illustrates schematically how the end-caps 501 can form a part of a membrane-type pumping device. The flexible end-cap lateral end-walls 502 are shown restrained by fixed support rods 531, which are attached to the elastic end-cap means by any suitable means 568. The filter housing 504 is caused to reciprocate along its longitudinal axis, for example by the action of a cam and shaft mechanism denoted generally by the numeral 535. The mechanism imparts a reciprocating motion to the filter housing via the sleeve 533, which is in fixed or frictional engagement with the housing 504. Valve means 534 are disposed along each of the arterial and venous bloodlines 539 and 540 respectively. The opening and closing of the said valves 534 is controlled by a control means (not shown) in synchronisation with the reciprocating filter housing 504. As can be seen from Figure 5a, during a first movement phase, in which the valve means 534 are closed, the housing 504 is moved in the direction indicated by the arrow C. By virtue of the movement of the housing 504 relative to the support rods 531, blood contained within the header chamber 512a, associated with the arterial blood line 539, is urged through the filter membranes 508, along the direction of arrows E into the header chamber 512b associated with the venous blood line 540. During a second phase of movement, illustrated at Figure 5b, valve means 534 are held in an open position, and the housing 504 is simultaneously moved in a second direction, indicated by the arrow C. The restraining action of the fixed support rods 531 is such that the end-cap associated with the arterial blood-line 539 is drawn outward relative to the housing, thereby causing liquid to be drawn into the chamber 512a through arterial blood line 539, and out of the chamber 512b into the venous blood line 539, as indicated by the arrows F₁ and F₂. Thereafter, the valve means 534 are again closed, and the filter housing is displaced in the first direction once again. It is to be understood that whilst these illustrations show the housing 504 being moved relative to fixed support rods 531, an alternative arrangement could be provided, in which actuation means are connected to the end-caps, with the housing held fixed. The pumping system described herein could equally be configured using only a single end-cap to form the membrane-type pump. The cam means 535 illustrated could be replaced by any suitable mechanism for imparting a reciprocating motion.

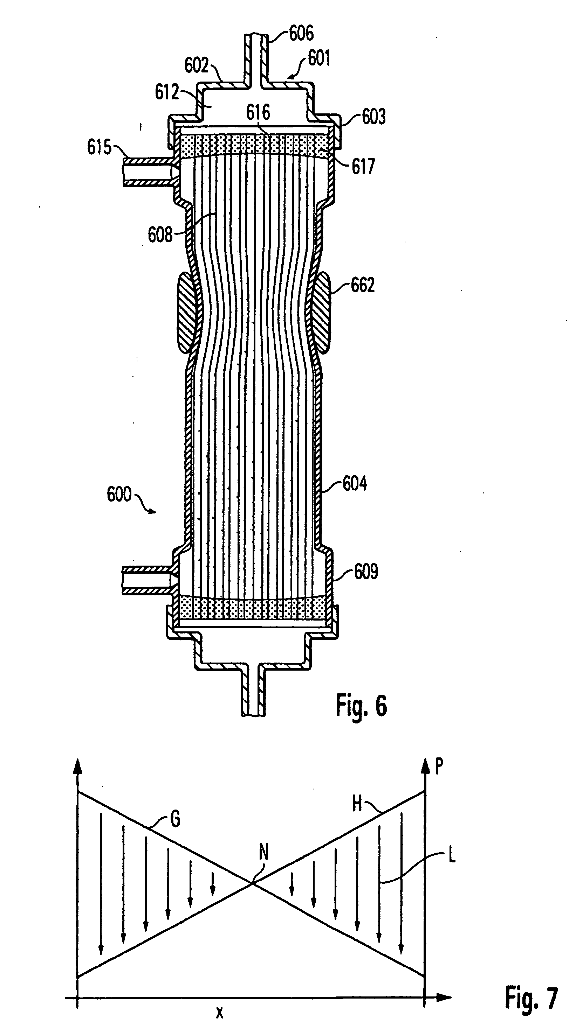

With regard to Figure 6, a development is shown (not claimed), according to which the filter housing 604 is made from a flexible elastic material. In this development, the housing is normally uniformly tubular, although its elastic properties allow it to be easily deformed, for example, using appropriate mechanical constricting means 662. The deformation in Figure 6 is shown in an exaggerated manner, purely for the purpose of illustration. If, for example, the tubular dialysis filter housing is constricted, as shown, during blood dialysis, the pressure profile of the respective fluids (blood and dialysis fluid) along the longitudinal axis of the filter housing may be changed. A typical transmembrane pressure profile is illustrated in Figure 7, in which the horizontal axis X represents the longitudinal distance along the filter, while the vertical axis P represents fluid pressure within the filter. The lines G and H plot the blood and dialysis or substitution fluid pressures respectively, at various points along the longitudinal axis of the filter. Migration of substances tends to occur from the higher-pressure liquid to the lower pressure liquid indicated by arrows L. By constricting the filter housing as shown, the intersection point N, at which the fluid pressures are equal, may be shifted to the right or left, thereby biasing the exchange of substances either out of the blood or out of the dialysis or substitution fluid; the zone in which one particular pressure differential exists, having been increased or decreased. It may be desirable, in order to obtain the desired relative pressure profile, to deform the housing 604 at more than one location along its longitudinal length.

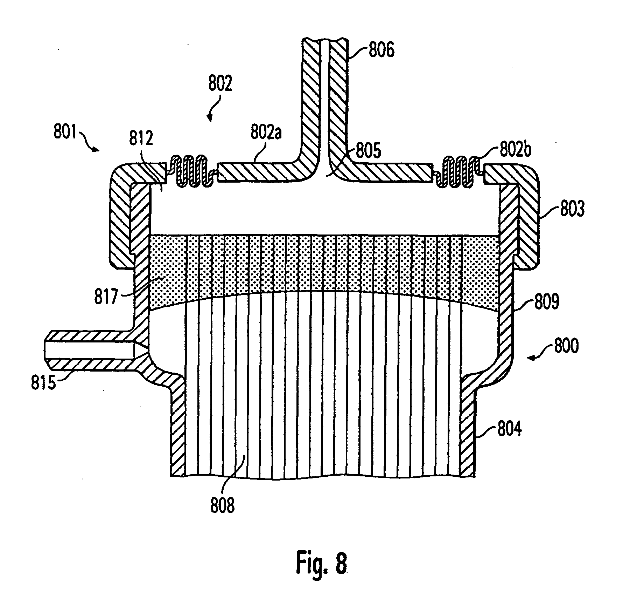

Figure 8 shows an end-cap 801 attached to a dialyser end portion 809 via attachment means 803. The end portion 809 of the dialyser filter housing 804 encloses the end portion of the filter means 808 and the securing means 817. The volume of the header chamber 812 is variable by virtue of the flexible portion 802b of the end-cap wall 802. A part 802a of the end-cap wall 802 is provided with an aperture 805, and a tubular portion 806, and is movable relative to the attachment means 803. The flexible portion 802b may be made from any suitable material for the intended purpose. In particular, it may comprise excess material for flexibility, as shown in Figure 8, or it may be made from suitable elastically deformable flexible material as already described.

According to the invention, any suitable amount of flexible material may be employed within the end-cap, from a comparatively limited extent, as shown in figure 8, to the entire end-cap as shown in Figures 1-6. Many alternative configurations may be employed, such as the provision of multiple flexible portions, or flexible portions having different thicknesses or shapes.

## Claims

1. End-cap (201, 301, 401, 501, 601, 801) for mounting on an end portion (309, 409, 509, 609, 809) of a dialyser (300, 400, 500, 600, 800), said end-cap (201, 301, 401, 501, 601, 801) comprising a wall (202, 302, 402, 502, 602, 802) defining an interior space (207) and at least one attachment portion (203, 303, 403, 603, 803) for attachment of said end-cap (201, 301, 401, 501, 601, 801 to said end portion (309, 409, 509, 609, 809), said wall (202, 302, 402, 502, 602, 802) further comprising an aperture (205, 305, 405, 605, 805) for liquid flow into or out of said interior space (207), **characterised in that** at least a part (802a) of said wall (202, 302, 402, 502, 602, 802), is movable relative to said attachment portion (203, 303, 403, 603, 803).

2. End-cap (201, 301, 401, 501, 601, 801) according to claim 1, wherein said at least a part (802a) of said wall (202, 302, 402, 502, 602, 802) including said aperture (205, 305, 405, 605, 805), is movable relative to said attachment portion (203, 303, 403, 603, 803) to change the volume of said interior space (207).

3. End-cap (201, 301, 401, 501, 601, 801) according to claim 1 or 2, wherein at least a portion (802b) of said end-cap is flexible, said flexible portion (802b) allowing movement of at least said part (802a) of said end-cap wall (202, 302, 402, 502, 602, 802) with respect to the said attachment portion (203, 303, 403, 603, 803).

4. End-cap (201, 301, 401, 501, 601, 801) according to claim 3, wherein said flexible portion (802b) of said end-cap wall (202, 302, 402, 502, 602, 802) is comprised of an excess material portion.

5. End-cap (201, 301, 401, 501, 601, 801) according to claim 3 or 4, wherein said flexible portion (802b) of the end-cap wall (202, 302, 402, 502, 602, 802) is comprised of flexible, elastic material.

6. End-cap (201, 301, 401, 501, 601, 801) according to any one of the preceding claims, said end-cap being made entirely from a flexible material having an elastic deformability of at least 5 %.

7. End-cap (201, 301, 401, 501, 601, 801) according to any one of claims 3-6, wherein said flexible material within said end-cap (201, 301, 401, 501, 601, 801) is comprised of a polymer with elastic properties.

8. End-cap (201, 301, 401, 501, 601, 801) according to any one of claims 3-7, wherein said flexible material within said end-cap (201, 301, 401, 501, 601, 801) is formed from a material consisting of polyurethane, polyvinylchloride, silicone based compounds or materials based on polypropylene and polyethylene which contain elastic polymers.

9. End-cap (201, 301, 401, 501, 601, 801) according to any one of claims 3-8, wherein said flexible material within said end-cap is comprised of a material having an elastic deformability of at least 10%.

10. End-cap (201, 301, 401, 501, 601, 801) according to any one of claims 3-9, wherein said flexible material within said end-cap is comprised of a material having an elastic deformability of at least 20%.

11. End-cap (201, 301, 401, 501, 601, 801) according to any one of claims 3-10, wherein said flexible material within said end-cap is comprised of a material having an elastic deformability between 10-30%.

12. End-cap (201, 301, 401, 501, 601, 801) according to any preceding claim, wherein said end-cap (201, 301, 401, 501, 601, 801) comprises an end wall (202, 302, 402, 502, 602, 802) and a peripheral wall (203, 303, 403, 603, 803), and wherein the end-wall is generally flat or conical and wherein the internal conical angle (α) lies between 0°- 30°.

13. End-cap according to claim 12, wherein said conical angle (α) lies between 7°-15°.

14. End-cap (201, 301, 401, 501, 601, 801) according to any preceding claim, additionally comprising a tubular portion (206, 306, 406, 606, 806) made integral with at least said movable part (802a) of said end-cap wall (202, 302, 402, 502, 602, 802), said tubular portion being aligned with said end-cap aperture (205, 305, 405, 605, 805) to define a flow path for transporting liquids, for example blood, through said end-cap aperture (205, 305, 405, 605, 805), into or out of said interior space (207).

15. End-cap (201, 301, 401, 501, 601, 801) according to claim 14, wherein said tubular portion (206, 306, 406, 606, 806) and at least said movable part (802a) of the end-cap wall (202, 302, 402, 502, 602, 802) are made from the same material in a single piece.

16. End-cap (201, 301, 401, 501, 601, 801) according to claim 14 or 15, wherein said tubular portion (206, 306, 406, 606, 806) forms at least a portion of a venous or arterial blood line (539, 540) for a dialysis system.

17. End-cap (201, 301, 401, 501, 601, 801) according to any one of claims 14 to 16, wherein said tubular portion (206, 306, 406, 606, 806) has a length between 0.5cm and several metres, measured from said end-cap wall (202, 302, 402, 502, 602, 802).

18. End-cap (201, 301, 401, 501, 601, 801) according to any one of the preceding claims, wherein said end-cap (201, 301, 401, 501, 601, 801) is formed transparent.

19. Dialyser (300, 400, 500, 600, 800) comprising a filter housing (204, 304, 404, 504, 604, 804) containing a semi-permeable filter means (208, 308, 408, 508, 608, 808) and at least one end-cap (201, 301, 401, 501, 601, 801) according to any preceding claim, wherein said end-cap is attached to an end (309, 409, 509, 609, 809) of the filter housing (204, 304, 404, 504, 604, 804) in a liquid-tight manner and wherein said interior space (207) forms a liquid inflow or outflow header chamber between the wall (202, 302, 402, 502, 802) of the said end-cap and the filter means (208, 308, 408, 508, 608, 808).

20. Dialyser (300, 400, 500, 600, 800) according to claim 19, wherein the end-cap (201, 301, 401, 501, 601, 801) is deformable in response to liquid pressure changes within said header chamber (212, 312, 412, 512, 812) and wherein means are arranged to detect the deformation of said end-cap (201, 301, 401, 501, 601, 801) caused by a change in liquid pressure within said header chamber (201, 301, 401, 501, 601, 801).

21. Dialyser (300, 400, 500, 600, 800) according to any one of the preceding claims 19-20, wherein said housing (304, 404, 504, 604, 804) is formed tubular and comprises said end-cap (201, 301, 401, 501, 601, 801) at each end, and wherein means for detecting arterial blood pressure are arranged on at least one of said end-caps (201, 301, 401, 501, 601, 801) and means for measuring venous blood pressure are arranged on the other said end-cap (201, 301, 401, 501, 601, 801).

22. Dialyser (300, 400, 500, 600, 800) according to any one of the preceding claims 20-21, wherein the means for detecting the deformation of an end-cap (201, 301, 401, 501, 601, 801) comprise a deformation sensor (470).

23. Dialyser (300, 400, 500, 600, 800) according to claim 22, wherein the deformation sensor comprises one or more strain measurement strips (470).

24. Dialyser (300, 400, 500, 600, 800) according to claim 22, wherein the deformation sensor comprises mechanical measurement means.

25. Dialyser (300, 400, 500, 600, 800) according to any one of claims 19-24, wherein at least one of said end-caps (501) is arranged to function as a liquid-pump for pumping blood through the filter means (508), said end-cap and dialyser being associated with means (535) for imparting a relative reciprocating movement between the said end-cap and the dialyser housing (504).

26. Dialyser (300, 400, 500, 600, 800) according to any one of claims 19-25, having an internal volume, which is variable such that the volume of blood, which may be accommodated within the dialyser filter means, can be varied.

27. Dialyser (300, 400, 500, 600, 800) according to any one of claims 19-26 wherein the end-caps (201, 301, 401, 501, 601, 801) are attached to the dialyser in a liquid-tight manner by ultrasound welding or by adhesive means.

28. Dialyser (300, 400, 500, 600, 800) according to any of claims 19-27, wherein the housing (204, 304, 404, 504, 604, 804) is formed tubular and wherein said semi-permeable filter means comprises a bundle of semi-permeable hollow fibre membranes (208, 308, 408, 508, 608, 808) and is arranged in the tubular housing.

29. Dialyser (300, 400, 500, 600, 800) according to one or more of the preceding claims 19-28, wherein at least one connection (315, 415, 515, 615, 815) is arranged on the filter housing (204, 304, 404, 504, 604, 804) for the removal and/or supply of fluid.

30. Method of removing gas bubbles from a liquid within a dialyser (300, 400, 500, 600, 800) as defined in any one of claims 19-29, whereby the end-cap (201, 301, 401, 501, 601, 801) is manipulated in such a manner that gas bubbles (460) collecting within an inflow or outflow header chamber (312, 412, 512, 612, 812) of the dialyser are directed out of said header chamber via the aperture (205, 305, 405, 805) in said wall (202, 302, 402, 502, 802) of the dialyser end-cap (201, 301, 401, 501, 601, 801).

31. System for pumping blood around an extracorporeal blood circuit, wherein the circuit includes a dialyser according to claim 19 and wherein means (535) are provided for imparting a relative reciprocating movement between the dialyser housing (304, 404, 504, 804) and a flexible wall portion of at least one end-cap (201, 301, 401, 501, 601, 801), said at least one end-cap thereby functioning as part of a membrane-type pump, capable of urging fluid though the dialyser filter (308, 408, 508, 808) and around a blood circuit.

## Patentansprüche

1. Endkappe (201, 301, 401, 501, 601, 801) zur Befestigung an einem Endabschnitt (309, 409, 509, 609, 809) eines Dialysators (300, 400, 500, 600, 800), wobei die Endkappe (201, 301, 401, 501, 601, 801) eine Wand (202, 302, 402, 502, 602, 802), die einen Innenraum (207) definiert, und zumindest einen Befestigungsabschnitt (203, 303, 403, 603, 803) zur Befestigung der Endkappe (201, 301, 401, 501, 601, 801) an dem Endabschnitt (309, 409, 509, 609, 809) umfasst, wobei die Wand (202, 302, 402, 502, 602, 802) ferner eine Öffnung (205, 305, 405, 605, 805) für einen Flüssigkeitsdurchfluss in oder aus dem Innenraum (207) umfasst, **dadurch gekennzeichnet, dass** zumindest ein Teil (802a) der Wand (202, 302, 402, 502, 602, 802) bezüglich des Befestigungsabschnitts (203, 303, 403, 603, 803) bewegbar ist.

2. Endkappe (201, 301, 401, 501, 601, 801) nach Anspruch 1, wobei der zumindest eine Teil (802a) der Wand (202, 302, 402, 502, 602, 802), die die Öffnung (205, 305, 405, 605, 805) umfasst, bezüglich des Befestigungsabschnitts (203, 303, 403, 603, 803) bewegbar ist, um das Volumen des Innenraums (207) zu ändern.

3. Endkappe (201, 301, 401, 501, 601, 801) nach Anspruch 1 oder 2, wobei zumindest ein Abschnitt (802b) der Endkappe flexibel ist, wobei der flexible Abschnitt (802b) eine Bewegung zumindest des Teils (802a) der Endkappenwand (202, 302, 402, 502, 602, 802) bezüglich des Befestigungsabschnitts (203, 303, 403, 603, 803) zulässt.

4. Endkappe (201, 301, 401, 501, 601, 801) nach Anspruch 3, wobei der flexible Abschnitt (802b) der Endkappenwand (202, 302, 402, 502, 602, 802) aus einem Abschnitt überschüssigen Materials besteht.

5. Endkappe (201, 301, 401, 501, 601, 801) nach einem der Ansprüche 3 oder 4, wobei der flexible Abschnitt (802b) der Endkappenwand (202, 302, 402, 502, 602, 802) aus einem flexiblen elastischen Material besteht.

6. Endkappe (201, 301, 401, 501, 601, 801) nach einem der vorhergehenden Ansprüche, wobei die Endkappe vollständig aus einem flexiblen Material gefertigt ist, das eine elastische Verformbarkeit von zumindest 5 % aufweist.

7. Endkappe (201, 301, 401, 501, 601, 801) nach einem der Ansprüche 3 bis 6, wobei das flexible Material in der Endkappe (201, 301, 401, 501, 601, 801) aus einem Polymer mit elastischen Eigenschaften besteht.

8. Endkappe (201, 301, 401, 501, 601, 801) nach einem der Ansprüche 3 bis 7, wobei das flexible Material in der Endkappe (201, 301, 401, 501, 601, 801) aus einem Material gebildet ist, das Polyurethan, Polyvinylchlorid, auf Silikon basierende Verbindungen oder Materialien auf Basis von Polypropylen und Polyethylen umfasst, die elastische Polymere aufweisen.

9. Endkappe (201, 301, 401, 501, 601, 801) nach einem der Ansprüche 3 bis 8, wobei das flexible Material in der Endkappe aus einem Material besteht, das eine elastische Verformbarkeit von zumindest 10 % aufweist.

10. Endkappe (201, 301, 401, 501, 601, 801) nach einem der Ansprüche 3 bis 9, wobei das flexible Material in der Endkappe aus einem Material besteht, das eine elastische Verformbarkeit von zumindest 20 % aufweist.

11. Endkappe (201, 301, 401, 501, 601, 801) nach einem der Ansprüche 3 bis 10, wobei das flexible Material in der Endkappe aus einem Material besteht, das eine elastische Verformbarkeit zwischen 10 - 30 % aufweist.

12. Endkappe (201, 301, 401, 501, 601, 801) nach einem der vorhergehenden Ansprüche, wobei die Endkappe (201, 301, 401, 501, 601, 801) eine Stirnwand (202, 302, 402, 502, 602, 802) und eine Umfangswand (203, 303, 403, 603, 803) umfasst und wobei die Stirnwand allgemein flach oder kegelförmig ist und wobei der innere Kegelwinkel (α) zwischen 0° - 30° liegt.

13. Endkappe nach Anspruch 12, wobei der Kegelwinkel (α) zwischen 7° - 15° liegt.

14. Endkappe (201, 301, 401, 501, 601, 801) nach einem der vorhergehenden Ansprüche, zusätzlich mit einem rohrförmigen Abschnitt (206, 306, 406, 606, 806), der einteilig mit zumindest dem bewegbaren Teil (802a) der Endkappenwand (202, 302, 402, 502, 602, 802) ausgebildet ist, wobei der rohrförmige Abschnitt mit der Endkappenöffnung (205, 305, 405, 605, 805) ausgerichtet ist, um einen Strömungspfad zum Transport von Flüssigkeiten, beispielsweise Blut, durch die Endkappenöffnung (205, 305, 405, 605, 805) in den Innenraum (207) hinein oder aus diesem heraus zu definieren.

15. Endkappe (201, 301, 401, 501, 601, 801) nach Anspruch 14, wobei der rohrförmige Abschnitt (206, 306, 406, 606, 806) und zumindest das bewegbare Teil (802a) der Endkappenwand (202, 302, 402, 502, 602, 802) aus demselben Material in einem einzelnen Stück gefertigt sind.

16. Endkappe (201, 301, 401, 501, 601, 801) nach einem der Ansprüche 14 oder 15, wobei der rohrförmige Abschnitt (206, 306, 406, 606, 806) zumindest einen Anteil einer Leitung (539, 540) für venöses oder arterielles Blut für ein Dialysesystem bildet.

17. Endkappe (201, 301, 401, 501, 601, 801) nach einem der Ansprüche 14 bis 16, wobei der rohrförmige Abschnitt (206, 306, 406, 606, 806) eine Länge gemessen von der Endkappenwand (202, 302, 402, 502, 602, 802) zwischen 0,5 cm und mehreren Metern besitzt.

18. Endkappe (201, 301, 401, 501, 601, 801) nach einem der vorhergehenden Ansprüche, wobei die Endkappe (201, 301, 401, 501, 601, 801) transparent ausgebildet ist.

19. Dialysator (300, 400, 500, 600, 800) mit einem Filtergehäuse (204, 304, 404, 504, 604, 804), das ein halbdurchlässiges Filtermittel (208, 308, 408, 508, 608, 808) und zumindest eine Endkappe (201, 301, 401, 501, 601, 801) nach einem der vorhergehenden Ansprüche umfasst, wobei die Endkappe an einem Ende (309, 409, 509, 609, 809) des Filtergehäuses (204, 304, 404, 504, 604, 804) in einer flüssigkeitsdichten Art und Weise befestigt ist, und wobei der Innenraum (207) eine Zufluss- oder Abflusssammelkammer für Flüssigkeit zwischen der Wand (202, 302, 402, 502, 802) der Endkappe und dem Filtermittel (208, 308, 408, 508, 608, 808) bildet.

20. Dialysator (300, 400, 500, 600, 800) nach Anspruch 19, wobei die Endkappe (201, 301, 401, 501, 601, 801) in Ansprechen auf Flüssigkeitsdruckänderungen in der Sammelkammer (212, 312, 412, 512, 812) verformbar ist, und wobei Mittel angeordnet sind, um die Verformung der Endkappe (201, 301, 401, 501, 601, 801), die durch eine Änderung des Flüssigkeitsdrucks in der Sammelkammer (201, 301, 401, 501, 601, 801) bewirkt wird, zu erfassen.

21. Dialysator (300, 400, 500, 600, 800) nach einem der vorhergehenden Ansprüche 19 - 20, wobei das Gehäuse (304, 404, 504, 604, 804) rohrförmig ausgebildet ist und die Endkappe (201, 301, 401, 501, 601, 801) an jedem Ende umfasst, und wobei Mittel zur Erfassung des arteriellen Blutdrucks an zumindest einer der Endkappen (201, 301, 401, 501, 601, 801) angeordnet sind und Mittel zum Messen des venösen Blutdrucks an der anderen Endkappe (201, 301, 401, 501, 601, 801) angeordnet sind.

22. Dialysator (300, 400, 500, 600, 800) nach einem der vorhergehenden Ansprüche 20 - 21, wobei die Mittel zum Erfassen der Verformung einer Endkappe (201, 301, 401, 501, 601, 801) einen Verformungssensor (470) umfassen.

23. Dialysator (300, 400, 500, 600, 800) nach Anspruch 22, wobei der Verformungssensor einen oder mehrere Dehnungsmessstreifen (470) umfasst.

24. Dialysator (300, 400, 500, 600, 800) nach Anspruch 22, wobei der Verformungssensor mechanische Messmittel umfasst.

25. Dialysator (300, 400, 500, 600, 800) nach einem der vorhergehenden Ansprüche 19 - 24, wobei zumindest eine der Endkappen (501) derart angeordnet ist, dass sie als eine Flüssigkeitspumpe zum Pumpen von Blut durch das Filtermittel (508) funktioniert, wobei die Endkappe und der Dialysator einem Mittel (535) zugeordnet sind, das eine relative hin- und herverlaufende Bewegung zwischen der Endkappe und dem Dialysatorgehäuse (504) ausübt.

26. Dialysator (300, 400, 500, 600, 800) nach einem der Ansprüche 19 - 25, mit einem Innenvolumen, das variabel ist, so dass das Blutvolumen, das in dem Dialysatorfiltermittel aufgenommen werden kann, variiert werden kann.

27. Dialysator (300, 400, 500, 600, 800) nach einem der Ansprüche 19 - 26, wobei die Endkappen (201, 301, 401, 501, 601, 801) an dem Dialysator in einer flüssigkeitsdichten Art und Weise durch Ultraschallschweißen oder durch Klebemittel angebracht sind.

28. Dialysator (300, 400, 500, 600, 800) nach einem der Ansprüche 19 - 27, wobei das Gehäuse (204, 304, 404, 504, 604, 804) rohrförmig ausgebildet ist und wobei das halbdurchlässige Filtermittel ein Bündel halbdurchlässiger Hohlfasermembrane (208, 308, 408, 508, 608, 808) umfasst und in dem rohrförmigen Gehäuse angeordnet ist.

29. Dialysator (300, 400, 500, 600, 800) nach einem der vorhergehenden Ansprüche 19 - 28, wobei zumindest eine Verbindung (315, 415, 515, 615, 815) an dem Filtergehäuse (204, 304, 404, 504, 604, 804) für die Entfernung und/oder Lieferung des Fluids angeordnet ist.

30. Verfahren zur Entfernung von Gasblasen von einer Flüssigkeit innerhalb eines Dialysators (300, 400, 500, 600, 800) nach einem der Ansprüche 19 - 29, wobei die Endkappe (201, 301, 401, 501, 601, 801) derart betätigt wird, dass Gasblasen (460), die sich in einer Zufluss- oder Abflusssammelkammer (312, 412, 512, 612, 812) des Dialysators ansammeln, aus der Sammelkammer über die Öffnung (205, 305, 405, 805) in der Wand (202, 302, 402, 502, 802) der Dialysatorendkappe (201, 301, 401, 501, 601, 801) heraus geführt werden.

31. System zum Pumpen von Blut um einen extrakorporalen Blutkreislauf herum, wobei der Kreislauf einen Dialysator nach Anspruch 19 umfasst und wobei Mittel (535) vorgesehen sind, um eine relative hin- und herverlaufende Bewegung zwischen dem Dialysatorgehäuse (304, 404, 504, 804) und einem flexiblen Wandabschnitt zumindest einer Endkappe (201, 301, 401, 501, 601, 801) auszuüben, wobei die zumindest eine Endkappe **dadurch** als Teil einer Pumpe vom Membrantyp funktioniert, die in der Lage ist, Fluid durch den Dialysatorfilter (308, 408, 508, 808) hindurch und um einen Blutkreislauf herum zu drängen.

## Revendications

1. Couvercle terminal (201, 301, 401, 501, 601, 801) pour monter sur une portion terminale (309, 409, 509, 609, 809) d'un dialyseur (300, 400, 500, 600, 800), ledit couvercle terminal (201, 301, 401, 501, 601, 801) comprenant une paroi (202, 302, 402, 502, 602, 802) définissant un espace intérieur (207) et au moins une portion d'attache (203, 303, 403, 603, 803) pour attacher ledit couvercle terminal (201, 301, 401, 501, 601, 801) sur ladite portion terminale (309, 409, 509, 609, 809), ladite paroi (202, 302, 402, 502, 602, 802) comprenant en outre une ouverture (205, 305, 405, 605, 805) pour l'écoulement d'un liquide entrant ou sortant dudit espace intérieur (207), **caractérisé en ce qu'**au moins une partie (802a) de ladite paroi (202, 302, 402, 502, 602, 802) est mobile par rapport à ladite portion d'attache (203, 303, 403, 603, 803).

2. Couvercle terminal (201, 301, 401, 501, 601, 801) selon la revendication 1, dans lequel ladite au moins une partie (802a) de ladite paroi (202, 302, 402, 502, 602, 802) incluant ladite ouverture (205, 305, 405, 605, 805) est mobile par rapport à ladite portion d'attache (203, 303, 403, 603, 803) pour changer le volume dudit espace intérieur (207).

3. Couvercle terminal (201, 301, 401, 501, 601, 801) selon la revendication 1 ou 2, dans lequel au moins une portion (802b) dudit couvercle terminal est flexible, ladite portion flexible (802b) permettant un mouvement de ladite partie au moins (802a) de ladite paroi (202, 302, 402, 502, 602, 802) de couvercle terminal par rapport à ladite portion d'attache (203, 303, 403, 603, 803).

4. Couvercle terminal (201, 301, 401, 501, 601, 801) selon la revendication 3, dans lequel ladite portion flexible (802b) de ladite paroi (202, 302, 402, 502, 602, 802) de couvercle terminal est constituée d'une portion de matériau en excès.

5. Couvercle terminal (201, 301, 401, 501, 601, 801) selon la revendication 3 ou 4, dans lequel ladite portion flexible (802b) de la paroi (202, 302, 402, 502, 602, 802) de couvercle terminal est constituée d'un matériau flexible élastique.

6. Couvercle terminal (201, 301, 401, 501, 601, 801) selon l'une quelconque des revendications précédentes, ledit couvercle terminal étant réalisé entièrement à partir d'un matériau flexible ayant une déformabilité élastique d'au moins 5 %.

7. Couvercle terminal (201, 301, 401, 501, 601, 801) selon l'une quelconque des revendications 3 à 6, dans lequel ledit matériau flexible dans ledit couvercle terminal (201, 301, 401, 501, 601, 801) est constitué d'un polymère avec des propriétés élastiques.

8. Couvercle terminal (201, 301, 401, 501, 601, 801) selon l'une quelconque des revendications 3 à 7, dans lequel ledit matériau flexible dans ledit couvercle terminal (201, 301, 401, 501, 601, 801) est formé d'un matériau constitué de composés à base de polyuréthane, chlorure de polyvinyle, silicone, ou matériaux basés sur du polypropylène et du polyéthylène qui contiennent des polymères élastiques.

9. Couvercle terminal (201, 301, 401, 501, 601, 801) selon l'une quelconque des revendications 3 à 8, dans lequel ledit matériau flexible dans ledit couvercle terminal est constitué d'un matériau ayant une déformabilité élastique d'au moins 10 %.

10. Couvercle terminal (201, 301, 401, 501, 601, 801) selon l'une quelconque des revendications 3 à 9, dans lequel ledit matériau flexible dans ledit couvercle terminal est constitué d'un matériau ayant une déformabilité élastique d'au moins 20 %.

11. Couvercle terminal (201, 301, 401, 501, 601, 801) selon l'une quelconque des revendications 3 à 10, dans lequel ledit matériau flexible dans ledit couvercle terminal est constitué d'un matériau ayant une déformabilité élastique entre 10 et 30 %.

12. Couvercle terminal (201, 301, 401, 501, 601, 801) selon l'une quelconque des revendications précédentes, dans lequel ledit couvercle terminal (201, 301, 401, 501, 601, 801) comprend une paroi terminale (202, 302, 402, 502, 602, 802) et une paroi périphérique (203, 303, 403, 503, 603, 803), et dans lequel ladite paroi terminale est généralement plate ou conique, et l'angle interne du cône (α) est compris entre 0° et 30°.

13. Couvercle terminal (201, 301, 401, 501, 601, 801) selon la revendication 12, dans lequel ledit angle du cône (α) est compris entre 7° et 15°.

14. Couvercle terminal (201, 301, 401, 501, 601, 801) selon l'une quelconque des revendications précédentes, comprenant additionnellement une portion tubulaire (206, 306, 406, 606, 806) réalisée de manière intégrale au moins avec ladite partie mobile (802a) de ladite paroi (202, 302, 402, 502, 602, 802) de couvercle terminal, ladite portion tubulaire étant alignée avec ladite ouverture (205, 305, 405, 605, 805) du couvercle terminal pour définir un passage d'écoulement pour transporter des liquides, par exemple du sang, à travers ladite ouverture (205, 305, 405, 605, 805) du couvercle terminal pour entrer ou pour sortir dudit espace intérieur (207).

15. Couvercle terminal (201, 301, 401, 501, 601, 801) selon la revendication 14, dans lequel ladite portion tubulaire (206, 306, 406, 606, 806) et au moins ladite partie mobile (802a) de la paroi (202, 302, 402, 502, 602, 802) de couvercle terminal sont réalisées du même matériau en une pièce unique.

16. Couvercle terminal (201, 301, 401, 501, 601, 801) selon la revendication 14 ou 15, dans lequel ladite portion tubulaire (206, 306, 406, 606, 806) forme au moins une portion d'une ligne de sang veineux ou artériel (539, 540) pour un système de dialyse.

17. Couvercle terminal (201, 301, 401, 501; 601, 801) selon l'une quelconque des revendications 14 à 16, dans lequel ladite portion tubulaire (206, 306, 406, 606, 806) a une longueur entre 0,5 cm et plusieurs mètres, mesurée de puis ladite paroi (202, 302, 402, 502, 602, 802) du couvercle terminal.

18. Couvercle terminal (201, 301, 401, 501, 601, 801) selon l'une quelconque des revendications précédents, dans lequel ledit couvercle terminal (201, 301, 401, 501, 601, 801) est formé transparent.

19. Dialyseur (300, 400, 500, 600, 800) comprenant un boîtier de filtre (204, 304, 404, 504, 604, 804) comprenant des moyens de filtrage semi-perméables (208, 308, 408, 508, 608, 808) et au moins un couvercle terminal (201, 301, 401, 501, 601, 801) selon l'une quelconque des revendications précédentes, dans lequel ledit couvercle terminal est attaché à une extrémité (309, 409, 509, 609, 809) du boîtier de filtre (204, 304, 404, 504, 604, 804) d'une manière étanche aux liquides, et dans lequel ledit espace intérieur (207) forme une chambre collectrice d'entrée ou de sortie entre la paroi (202, 302, 402, 502, 602, 802) dudit couvercle terminal et les moyens de filtrage (208, 308, 408, 508, 608, 808).

20. Dialyseur (300, 400, 500, 600, 800) selon la revendication 19, dans lequel le couvercle terminal (201, 301, 401, 501, 601, 801) est déformable en réponse aux changements de la pression du liquide dans ladite chambre collectrice (212, 312, 412, 512, 812), et dans lequel des moyens sont agencés pour détecter la déformation dudit couvercle terminal (201, 301, 401, 501, 601, 801) provoquée par un changement de la pression du liquide dans ladite chambre collectrice (212, 312, 412, 512, 812).

21. Dialyseur (300, 400, 500, 600, 800) selon l'une quelconque des revendications 19 à 20, dans lequel ledit boîtier (204, 304, 404, 504, 604, 804) est formé tubulaire et comprend ledit couvercle terminal (201, 301, 401, 501, 601, 801) à chaque extrémité, et dans lequel des moyens pour détecter la pression sanguine artérielle sont agencés sur l'un au moins desdits couvercles terminaux (201, 301, 401, 501, 601, 801), et des moyens pour mesurer la pression sanguine veineuse sont agencés sur l'autre couvercle terminal (201, 301, 401, 501, 601, 801).

22. Dialyseur (300, 400, 500, 600, 800) selon l'une quelconque des revendications précédentes 20 et 21, dans lequel les moyens pour détecter la déformation d'un couvercle terminal (201, 301, 401, 501, 601, 801) comprennent un capteur de déformation (470).

23. Dialyseur (300, 400, 500, 600, 800) selon la revendication 22, dans lequel le capteur de déformation comprend une ou plusieurs jauges de contraintes (470).

24. Dialyseur (300, 400, 500, 600, 800) selon la revendication 22, dans lequel le capteur de déformation comprend des organes de mesure mécaniques.

25. Dialyseur (300, 400, 500, 600, 800) selon l'une quelconque des revendications 19 à 24, dans lequel l'un au moins desdits couvercles terminaux (501) est agencé pour fonctionner comme pompe à liquide afin de pomper du sang à travers les moyens de filtrage (508), ledit couvercle terminal et ledit dialyseur étant associés à des moyens (535) pour imposer un mouvement de va-et-vient relatif entre ledit couvercle terminal et le boîtier (504) du dialyseur.

26. Dialyseur (300, 400, 500, 600, 800) selon l'une quelconque des revendications 19 à 25, ayant un volume interne qui est variable, de telle sorte que le volume de sang qui peut être reçu dans les moyens de filtrage du dialyseur peut être varié.

27. Dialyseur (300, 400, 500, 600, 800) selon l'une quelconque des revendications 19 à 26, dans lequel les couvercles terminaux (201, 301, 401, 501, 601, 801) sont attachés au dialyseur d'une manière étanche aux liquides par soudage aux ultrasons ou par des moyens adhésifs.

28. Dialyseur (300, 400, 500, 600, 800) selon l'une quelconque des revendications 19 à 27, dans lequel le boîtier (204, 304, 404, 504, 604, 804) est formé tubulaire, et dans lequel lesdits moyens de filtrage semiperméables comprennent un faisceau de membranes à fibres creuses semiperméables (208, 308, 408, 508, 608, 808) et sont agencés dans le boîtier tubulaire.

29. Dialyseur (300, 400, 500, 600, 800) selon l'une ou plusieurs des revendications 19 à 28, dans lequel au moins une connexion (215, 315, 415, 515, 815) est agencée sur le boîtier de filtre (204, 304, 404, 504, 604, 804) pour l'enlèvement et/ou l'alimentation de fluide.

30. Procédé pour enlever des bulles de gaz à partir d'un liquide dans un dialyseur (300, 400, 500, 600, 800) tel que défini dans l'une quelconque des revendications 19 à 29, dans lequel le couvercle terminal (201, 301, 401, 501, 601, 801) est manipulé de telle manière que les bulles de gaz (460) qui se collectent dans une chambre collectrice d'entrée ou de sortie (212, 312, 412, 512, 812) du dialyseur sont dirigées hors de ladite chambre collectrice via l'ouverture (205, 305, 405, 605, 805) dans ladite paroi (202, 302, 402, 502, 602, 802) du couvercle terminal (201, 301, 401, 501, 601, 801) du dialyseur.

31. Système pour pomper du sang autour d'un circuit sanguin extracorporel, dans lequel le circuit inclut un dialyseur selon la revendication 19, et dans lequel sont prévus des moyens (535) pour imposer un mouvement de va-et-vient relatif entre le boîtier (204, 304, 404, 504, 604, 804) du dialyseur et une portion de paroi flexible d'au moins un couvercle terminal (201, 301, 401, 501, 601, 801), ledit au moins un couvercle terminal fonctionnant ainsi en tant que partie d'une pompe du type à membrane, capable de chasser le fluide à travers le filtre (308, 408, 508, 808) du dialyseur et autour d'un circuit sanguin.
